# EUROPÄISCHE PATENTANMELDUNG

(11) **EP 1 336 659 A2**
(43) Veröffentlichungstag der Anmeldung: **20.08.2003**
(21) Anmeldenummer: 03002774.2
(22) Anmeldetag: 07.02.2003
(51) Int. Cl.: C12P 7/64, C12P 7/62, C12P 7/04, A61K 7/00, A61K 31/215

(54) **Verfahren zur Gewinnung von Hydroperoxiden**

(30) Priorität: 16.02.2002 DE 10206504
(71) Anmelder: Cognis Deutschland GmbH & Co. KG, 40589 Düsseldorf (DE)
(72) Erfinder: Weiss, Albrecht, Dr., 40764 Langenfeld (DE); Schörken, Ulrich, Dr., 40591 Düsseldorf (DE); Candar, Volkan, Dr., 81464 Tuzla, Istanbul (TR); Eryasa, Yonca, 81120 Icerenkoy, Istanbul (TR); Wunderlich, Markus, 40789 Monheim (DE); Buyukuslu, Nihal, Kartal, Istanbul (TR); Beverungen, Carsten, 40591 Düsseldorf (DE)

(57) **Zusammenfassung**

Vorgeschlagen wird ein Verfahren zum Gewinnen von Hydroperoxiden aus einem nativen Fett oder Öl enthaltend ungesättigte Fettsäuren, oder ungesättigte Fettsäuren oder deren Derivate und/oder deren Mischungen,, dadurch gekennzeichnet, dass das native Fett oder Öl enthaltend ungesättigte Fettsäuren, oder ungesättigte Fettsäuren oder deren Derivate und/oder deren Mischungen, unter katalytischer Einwirkung von Lipoxidase in Gegenwart von Katalase bei einer Temperatur zwischen 5 und 50 °C peroxidiert werden, wobei die Katalase aus zudosiertem Wasserstoffperoxid Sauerstoff zur Peroxidation in situ produziert und die wässrige Phase mit den Enzymen von der Ölphase gegebenenfalls abgetrennt wird und das so erhaltene Produkt gegebenenfalls chemisch transformiert und konfektioniert wird.

## Beschreibung

### Gebiet der Erfindung

Die Erfindung befindet sich auf dem Gebiet der Hydroperoxidierung von nativen Ölen, Fetten sowie Estem, die ungesättigte Fettsäuren enthalten und die freien Fettsäuren und betrifft ein Verfahren zum Gewinnen von Hydroperoxiden und die Verwendung der nach diesem Verfahren hergestellten Hydroperoxide.

### Stand der Technik

Hydroperoxide sind organische Verbindungen der allgemeinen Formel R-O-OH; die Benennung erfolgt durch Voranstellen des Präfixes Hydroperoxy-. Die Hydroperoxide treten häufig bei der Autoxidation und bei der Reaktion von Organometall-Verbindungen (z. B. Grignard-Verbindungen) mit Sauerstoff auf, die Bildung (Oxygenierung) ist in beiden Fällen meist die Folge radikalischer Prozesse.

Peroxidierte und hydroperoxidierte Fettsäuren und deren Ester oder Glyceride sind Zwischenstufen in biologischen Stoffwechselwegen von Pflanzen und Säugetieren zur Synthese von bioaktiven Substanzen, die der Schädlingsabwehr dienen bzw. an Inflammationsvorgängen beteiligt sind. Die Bildung der Peroxide und Hydroperoxide wird in diesen Pflanzen und Säugetieren von Lipoxidasen oder Cyclooxygenasen katalysiert.

In Säugetieren sind peroxidierte Fettsäuren und ihre Folgeprodukte massgeblich an Inflammationsprozessen beteiligt. Der Entzündungsvorgang unterliegt einer komplexen Regulation, wobei pro- und antiinflammatorische Mediatoren wirken. In der Haut antiinflammatorisch wirkende Substanzen wie 13-Hydroxyoctadecadiensäure (Coriolsäure, 13-HODE) und 15-Hydroxyeicosatetraensäure (15-HETE) werden über 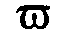 6-Lipoxidase Peroxidation gebildet. Der Einsatz von 13-HODE in kosmetischen Formulierungen mit anti-psoriatischer Wirkung ist beschrieben in der WO 99/44585.

Von Martini et al. konnte gezeigt werden, dass die Synthese von 13-HODE mit pflanzlichen Lipoxidasen (z.B. aus Soja) möglich ist (Martini D. et al., 1994, Optimization of large scale preparation of 13-(S)-hydroperoxy-9Z,11E-octadecadienoic acid using soybean lipoxygenase: application to the chemoenzymatic synthesis of (+)-coriolic acid, Biocatalysis 11, 47-63).

In pflanzlichen Systemen wird über die Synthese von peroxidierten Fettsäuren die Bildung von Phytooxylipinen eingeleitet, die der Pflanzenabwehr gegenüber Schädlingen dient (E. Blee; Biosynthesis of phytooxylipins: the peroxygenase pathway, (1998), Fett/Lipid 100, 121-127). Über enzymatische Biotransformation mit Lipoxidasen wurden bisher insbesondere die Aromakomponenten Hexenal, Hexenol sowie Nonadienal und Nonadienol hergestellt (WO9324644, EP0911414). Die Alkohole werden dabei in einem dreistufigen Verfahren gewonnen. Das mit Lipoxidase hergestellte 13-Hydroperoxid wird über Hydroperoxid-Lyase gespalten. Als Hydroperoxid-Lyase-Quelle wird ein Pflanzenextrakt eingesetzt. Das so entstandene Produkt Hexanal wird dann über Hefe-Reduktion zu Hexenol biotransformiert.

Lipoxidasen zählen zu den Oxidoreduktasen und gehören zu den Lipid-abbauenden Enzymen, die die Oxidation mehrfach ungesättigter Fettsäuren katalysieren und besonders in Sojabohnen (EC 1.13.11.12) und den Vorläufern der Erythrocyten, den Reticulocyten (EC 1.13.11.33, 15-L.), vorkommen Die in den letzteren in großen Mengen produzierte Lipoxidase greift auch komplexe Lipid-Moleküle an und dient zum Abbau der intrazellulären Membranen bei der Differenzierung zum reifen Erythrocyten. Die Umwandlung der Arachidonsäure in Leukotriene beginnt in Leukocyten mit der Einwirkung einer 5-Lipoxygenase (EC 1.13.11.34). Daneben ist eine 12-Lipoxidase (EC 1.13.11.31, in Thrombocyten) bekannt, die Arachidonsäure zu 12-Hydroperoxyeicosatetraensäure umsetzt. Lipoxidasen sind zudem auch in vielen Bakterien und Pilzen nachgewiesen worden. Lipoxidasen enthalten Eisen- und Zink-lonen.

Im Sauren konnte die Synthese von 9-Hydroperoxid mit Sojabohnen-Lipoxidase durchgeführt werden (Hiruta O. et al., Production of 9-hydroperoxy-gamma-linolenic acid by soybean lipoxygenase in a two-phase system, 1988, JAOCS 65, 1911-1914). Die meisten Arbeiten wurden allerdings zur Synthese des 13-Hydroperoxid durchgeführt. Dabei wird zumeist ein Lösungsmittel oder ein Detergenz zur Lösungsvermittlung zum Reaktionsansatz zugegeben (Piazza G.J. et al, 1996, Soybean lipoxygenase promoted oxidation of free and esterified linoleic acid in the presence of deoxycholate, JAOCS 73, 1045-1049).

Die Katalase (EC 1.11.1.6) ist ein Enzym, das in pflanzlichen und tierischen Geweben allgemein verbreitet ist und Wasserstoffperoxid in Wasser und Sauerstoff zerlegt:

Bei aeroben (Sauerstoff-verbrauchenden) Stoffwechselprozessen entsteht unter der Einwirkung von Oxidasen in besonderen Zellorganellen (Peroxisomen) Wasserstoffperoxid, das von der gleichzeitig anwesenden Katalase zersetzt wird (Schutzfunktion). Chemisch ist Katalase ein tetrameres Eisen-Protein mit MR 245 000 mit 4 Häm-Molekülen in Form von Ferriprotoporphyrin IX mit Eisen(III). Katalase ist eines der "schnellsten" Enzyme mit einer spezifischen Aktivität von 0,08 kat (5 · 106 U) pro mmol. Hemmstoffe sind z. B. Schwefelwasserstoff, Blausäure, Fluoride, Azide und Hydroxylamin. Katalase wird beispielsweise zur Zersetzung von Wasserstoffperoxid verwendet, welches bei der Sterilisierung von Milch zugesetzt wurde.

Bei herkömmlichen Verfahren wird der benötigte Sauerstoff zur Hydroperoxidierung durch Einblasen von Luft oder Sauerstoff in das Reaktionsgemisch zur Verfügung gestellt. Die in der Literatur beschriebenen Verfahren zur Hydroperoxiderung oder Peroxidierung verwenden zumeist Detergenzien oder Lösungsmittel zur besseren Emulgierung von Wasser- und Ölphase, um eine ausreichende Reaktion der wasserlöslichen Lipoxidase am Öl zu gewährleisten.

Es wird kein Hinweis darauf gegeben, wie es möglich wird, eine Peroxidierung mit Lipoxidasen ohne Lösungsmittel bei gleichzeitiger Durchmischung des Wasser- Öl Gemisches zu erreichen.

### Beschreibung der Erfindung

Die Aufgabe der vorliegenden Patentanmeldung hat darin bestanden, ein großtechnisches Verfahren zu entwickeln, mit dem es möglich wird, effektiv, wirtschaftlich, in wenigen Reaktionsschritten unter weitgehender Vermeidung toxikologisch und ökologisch bedenklicher Reaktionsschritte Hydroperoxide in hohen Ausbeuten und mit hoher Reinheit aus einem nativen Fett oder Öl herzustellen. Eine weitere Aufgabe der vorliegenden Patentanmeldung hat darin bestanden, die nach dem erfindungsgemäßen Verfahren hergestellten Hydroperoxide in den unterschiedlichsten Gebieten zur Anwendung zu bringen um ihre Herstellung wirtschaftlich zu machen.

Gegenstand der Erfindung ist ein Verfahren zum Gewinnen von Hydroperoxiden aus einem nativen Fett oder Öl enthaltend ungesättigte Fettsäuren, oder ungesättigte Fettsäuren oder deren Derivate und/oder deren Mischungen, dadurch gekennzeichnet, dass
a) das native Fett oder Öl enthaltend ungesättigte Fettsäuren, oder ungesättigte Fettsäuren oder deren Derivate und/oder deren Mischungen unter katalytischer Einwirkung von Lipoxidase in Gegenwart von Katalase bei einer Temperatur zwischen 5 und 50°C peroxidiert werden, wobei die Katalase aus zudosiertem Wasserstoffperoxid Sauerstoff zur Peroxidation in situ produziert und
b) die wässrige Phase mit den Enzymen von der Ölphase gegebenenfalls abgetrennt wird
c) das so erhaltene Produkt gegebenenfalls chemisch transformiert und gegebenenfalls konfektioniert wird.

Es wurde überraschenderweise gefunden, dass Hydroperoxide in einem Multienzymverfahren unter Einwirkung von Lipoxygenase und Katalase aus nativen Fett oder Öl enthaltend ungesättigte Fettsäuren, oder ungesättigte Fettsäuren oder deren Derivate und/oder deren Mischungen hergestellt werden können. Bevorzugt ist die Herstellung von niedrigviskosen Hydroperoxiden. Für Fischöl bedeutet dies bei einer Anfangsviskosität von 98 cps eine Endviskosität nach erfolgter Hydroperoxidierung im Bereich von 100 bis 150 cps, bevorzugt eine Viskosität im Bereich von 100 bis 120 cps.

Erfindungswesentlich ist die Kombination aus Katalase zur in situ Erzeugung von Sauerstoff mit Lipoxidase zur Addition des Sauerstoffs an ungesättigte Fettsäuren und/oder deren Derivaten zur Herstellung von Hydroperoxiden.

Ein Vorteil des erfindungsgemäßen Verfahrens ist es, dass keine Zugabe von Lösungsvermittlem nötig ist. Die Erzeugung des mikrofeinen Sauerstoffs in Lösung fördert die ausreichende Bildung einer Emulsion, so dass die Hydroperoxidierung stattfindet.

Weitere Vorteile des erfindungsgemäßen Verfahrens sind beispielsweise, dass die enzymatische Peroxidierung bei Raumtemperatur ohne das Einblasen von Luft oder Sauerstoff erfolgt, da Sauerstoff in situ erzeugt wird. Dadurch wird in der enzymatischen Reaktion Energie eingespart (Heizung, Gebläse) und Gerüche, die durch das Heissblasen insbesondere von Fischöl freigesetzt werden, werden vermieden.

Durch die milden Reaktionsbedingungen entstehen in der enzymatischen Hydroperoxidierung kaum Nebenprodukte, so wird bei Peroxidzahlen von > 500 noch keine erhöhte Viskosität im Öl detektiert. Die Anzahl der Doppelbindungen bleibt während der enzymatischen Peroxidierung konstant (detektiert über lodzahl-Messungen), da in der enzymatischen Reaktion Doppelbindungen nur geshiftet, nicht aber oxidiert werden. Der konstante Wert der lod-Zahl beweist, dass die Doppelbindungen während der enzymatischen Peroxidierung nicht durch Nebenreaktionen umgesetzt werden. Im Gegensatz zur chemischen Peroxidierung wird in der enzymatischen Reaktion ein einheitliches selektiv transformiertes Produkt gebildet, was in Nachbarschaft zur Hydroperoxidgruppe ein konjugiertes Doppelbindungssystem aufweist, wenn mehrfach ungesättigte Fettsäuren hydroperoxidiert werden.

Im Sinne der Erfindung sind unter Lipoxidasen solche Enzyme zu verstehen, die in der Lage sind, ungesättigte Fettsäuren zu Hydroperoxiden oder Peroxiden umzusetzen.

Lipoxidasen katalysieren die oxidative Addition von Sauerstoff an mehrfach ungesättigte Fettsäuren wie z.B. Linolsäure. Die Enzyme besitzen ein Eisenkation im aktiven Zentrum, das an der Katalyse beteiligt ist. Sauerstoff wird vom Enzym aktiviert und kann an einer Doppelbindung der ungesättigten Fettsäure angreifen. Durch die Anlagerung des Sauerstoffs kommt es zu einem Shift der Doppelbindung, wodurch im Falle mehrfach ungesättigter Fettsäuren ein Hydroperoxid mit konjugiertem Doppelbindungssystem entsteht.

Lipoxidasen wurden in verschiedenen Pflanzen (Fauconnier M.-L. et al., Assessment of lipoxygenase activity in seed extracts from 35 plant species; 1995, Grasas y Aceites 46, 6-10) sowie in Säugetieren und Mikroorganismen nachgewiesen. Für pflanzliche Lipoxidasen wurde die Möglichkeit der rekombinanten mikrobiellen Herstellung beschrieben (Hughes R.K. et al., 1999, Recombinant pea and potato lipoxygenases: versatile biocatalysts for industry, Agro-Food-Ind. Hi-Tech 10, 3-6).

Verschiedene Typen von Lipoxidasen existieren, die sich sowohl in ihrem Substratspektrum als auch in ihrer Regioselektivität unterscheiden. Natürliche Substrate pflanzlicher Lipoxidasen sind Linol- und Linolensäure, die Bestandteil vieler pflanzlicher Öle sind. Unterschieden werden dabei Lipoxidasen, die entweder das 9- oder das 13-Hydroperoxid bilden. Höhere tierische Organismen besitzen eine Vielzahl verschiedener Lipoxidase unterschiedlicher Spezifität. Substrate dieser Lipoxidasen sind neben Linol- und Linolensäure hauptsächlich höher ungesättigte Fettsäuren wie z.B. Arachidonsäure.

Besonders bevorzugt im Sinne der Erfindung werden Lipoxidasen eingesetzt, die aus Sojabohnen und/oder aus Mikroorganismen und/oder über rekombinante Expression aus Mikroorganismen gewonnen werden. Zur Herstellung von Lipoxidasen die rekombinant in Mikroorganismen exprimiert werden, kann prinzipiell jedes geeignete Verfahren genutzt werden, beispielhaft wird auf das Verfahren in Hughes R.K. et al., 1999, Recombinant pea and potato lipoxygenases: versatile biocatalysts for industry, Agro-Food-Ind. Hi-Tech 10, 3-6 verwiesen. Sojabohnen besitzen 3 Lipoxidase-Isoenzyme, die sich in ihrem pH-Optimum und in ihrer Produkspezifität unterscheiden. LOX-1 ist aktiv im leicht alkalischen und besitzt eine erhöhte Spezifität für die Bildung des 13-Hydroperoxids, wogegen die beiden Isoenzyme LOX-2 und LOX-3 aktiv im schwach alkalischen sind und gemischte 9- und 13-Hydroperoxide bilden. Die Isoenzyme sind chromatographisch zu trennen. Insbesondere bevorzugt ist der direkte Einsatz von gemahlenen Sojabohnen in wässriger Lösung als Quelle des Enzyms, welches direkt zum nativen Fett oder Öl addiert wird. Durch Zugabe des protein- und enzymhaltigen Sojamehls wird das Öl-Wasser Reaktionsgemisch gut emulgiert, wodurch die enzymatische Aktivität verbessert wird.

Synonym zum Begriff Lipoxidase wird im Sinne der Erfindung der Begriff Lipoxygenase verwendet.

Im Sinne der Erfindung sind Katalasen Enzyme, die in der Lage sind, Wasserstoffperoxid in Wasser und Sauerstoff zu zerlegen. Katalasen sind in tierischen und pflanzlichen Geweben allgemein verbreitet, sie kommen auch in Mikroorganismen vor. Erfindungsgemäß werden in einer weiteren Ausführungsform bevorzugt Katalasen aus Mikroorganismen, insbesondere aus Aspergillus niger und/oder Micrococcus lysodeikticus eingesetzt.

In einer bevorzugten Ausführungsform des erfindungsgemäßen Verfahrens sind die eingesetzten Derivate ungesättigter Fettsäuren Ester dieser Fettsäuren, insbesondere die Methyl und/oder Ethylester und/oder Glyceride.

Unter dem Begriff natives Fett oder Öl sind im Sinne der Erfindung alle Fette und Öle zu verstehen, die einen Gehalt an mehrfach ungesättigten Fettsäuren und/oder deren Derivate besitzen wobei "mehrfach ungesättigt" im Sinne der Erfindung eine Anzahl Doppelbindungen in einer Fettsäure größer oder gleich zwei bedeutet und diese konjugiert und/oder nicht konjugiert vorliegen können. Im Besonderen handelt es sich um Fischöl, Sonnenblumenöl, Tallöl, Leinöl, Sesamöl, Sojaöl, Mohnöl, Mandelöl, Maiskeimöl, Palmöl, Rapsöl, Reiskeimöl, Erdnussöl oder Baumwollsaatöl. Bei den bevorzugten Fettsäuren handelt es sich um Linolsäure, Linolensäure, Arachidonsäure, Docosahexaensäure, Elaeostearinsäure, Sorbinsäure und Clupanodonsäure, wobei Linolsäure, Linolensäure und/oder Arachidonsäure besonders bevorzugt sind. Targets für die enzymatische Hydroperoxidierung von Fetten und Ölen, insbesondere von Fischöl sind bevorzugt die in Triglyceriden gebundene Linol- und Linolensäure sowie die höher ungesättigten Fettsäuren basierend auf C20- und C22-Fettsäuren.

### Reaktionsschritt a)

Die erfindungsgemäßen Reaktionsbedingungen im Reaktionschritt a) der enzymatischen Katalyse richten sich nach dem optimalen Reaktionsbereich der ausgewählten Enzyme. Im Besonderen handelt es sich um Bedingungen bei denen unter anderem die Reaktionstemperatur zwischen 5 und 50 °C, bevorzugt eine Temperatur zwischen 10 und 30 °C gewählt wird.

Die erfindungsgemäß einzusetzenden Enzyme können in unterschiedlichen Formen eingesetzt werden. Prinzipiell sind alle dem Fachmann gebräuchlichen Darreichungsformen von Enzymen anwendbar. Vorzugsweise werden die Enzyme in reiner Form oder als technisches Enzympräparat entweder immobilisiert und/oder in Lösung, insbesondere in wässriger Lösung der ein Puffer zugesetzt wurde eingesetzt. Insbesondere bevorzugt ist der direkte Einsatz von gemahlenen Sojabohnen in wässriger Lösung bzw. ein filtrierter wässriger Extrakt der Sojabohnen als Quelle des Enzyms.

Erfindungsgemäß wird 30-%ige Wasserstofperoxidlösung kontinuierlich dem Reaktionsgemisch enthaltend Lipoxidase, Katalase, Fett oder Öl enthaltend ungesättigte Fettsäuren, oder ungesättigte Fettsäuren oder deren Derivate zugepumpt. Durch das kontinuierliche Zupumpen der Wasserstoffperoxidlösung wird das Reaktionsgemisch durchmischt, so dass kein Lösungsmittel zugesetzt werden muss. Der Zufluss der Wasserstoffperoxidlösung wird bevorzugt so eingestellt, daß der Gehalt an H₂O₂ in der wässrigen Lösung unterhalb von 2 mg/l liegt und daß keine Schaumbildung durch gebildetes O₂ erfolgt, d.h. die O₂ Bildung wird auf den O₂-Verbrauch durch die Lipoxidase angepasst.

### Reaktionsschritt b)

In einem zweiten Reaktionsschritt kann das Hydroperoxid gegebenenfalls vom verwendeten Multienzym aus Lipoxidase und Katalase abgetrennt werden. Prinzipiell sind zur Abtrennung der verwendeten Enzymkatalysatoren alle möglichen Trennverfahren einsetzbar, mit denen die genannten Enzymkatalysatoren abtrennbar sind. Besonders bevorzugt ist die Abtrennung durch eine Phasenseperation. In einer möglichen Ausführungsform handelt es sich bei dem Trennverfahren um Zentrifugieren oder Separation über einen Tellerseparator.

### Reaktionsschritt c)

In einem letzten Verfahrensschritt kann das so erhaltene Produkt gegebenenfalls chemisch transformiert und anschliessend konfektioniert werden. Die Hydroperoxide werden bis zur weiteren chemischen Umsetzung bevorzugt gekühlt gelagert, insbesondere bei Temperaturen zwischen -30 und 10 °C.

Die vorliegende Erfindung schließt die Erkenntnis ein, dass durch die Kombination von Lipoxygenase und Katalase in der Reaktionslösung ein Verfahren entwickelt wurde, mit dem es möglich ist, wirtschaftlich und ökologisch aus nativen Fetten und/oder Ölen, mehrfach ungesättigten Fettsäuren oder deren Derivate Hydroperoxide mit hoher Reinheit herzustellen.

Aus der Reaktion von Sojabohnen-Lipoxidase mit Triglyceriden aus Fischöl konnten aus den Edukten Linolsäureester, Eicosatraensäureester und Docosahexaensäureester beispeilhaft folgende Hydroperoxide durch das erfindungsgemäße Verfahren hergestellt werden:
Linolsäureester (Edukt) 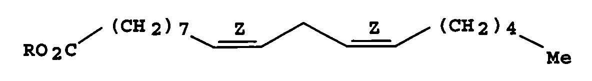
Peroxidierter Linolsäureester (Produkt) 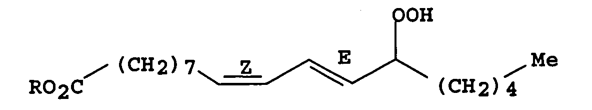
Eicosatetraensäureester (Edukt) 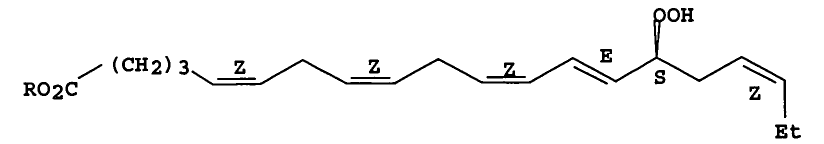
Peroxidierter Eicosatetraensäureester (Produkt) 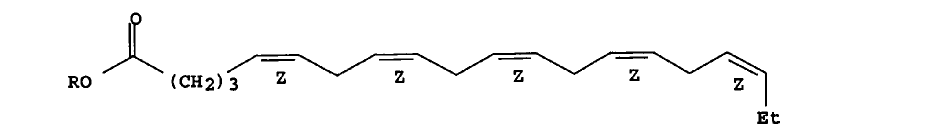
Docosahexaensäureester (Edukt) 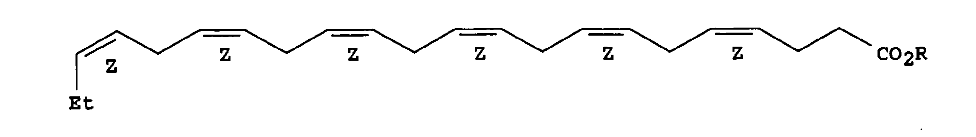
Peroxidierter Docosahexaensäureester (Produkt)

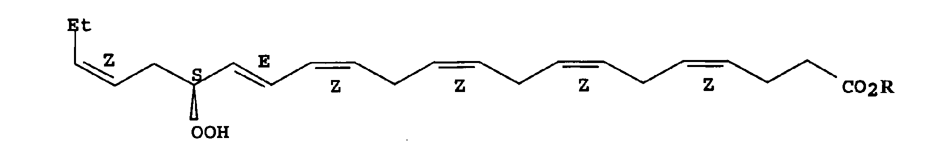

Ein weiterer Gegenstand der Erfindung betrifft ein Verfahren zur Herstellung von Sulfonaten zur Lederfettung, dadurch gekennzeichnet, dass Hydroperoxide, hergestellt nach dem erfindungsgemäßen Verfahren, anschliessend sulfoniert werden durch Reaktion mit Sulfiermitteln ausgewählt aus der Gruppe, die gebildet wid aus Schwefeltrioxid, Natriumbisulfit, Schwefelsäure, Oleum, Chlorsulfonsäure und Amidosulfonsäure. Die bevorzugten Hydroperoxide für die Sulfonierung stellen die enzymatisch hergestellten Hydroperoxide aus Ölen mit mehrfach ungesättigten Fettsäuren, bevorzugt Fischöl dar, die durch Reaktion mit Lipoxidasen aus zugesetztem gemahlenen Sojabohnen nach oben beschriebenen Verfahren hergestellt werden. In einer bevorzugten Ausführungsform wird eine Peroxidzahl im Bereich von 100 bis 300 im Triglycerid eingestellt. Die sich anschliessende Sulfonierung des enzymatisch peroxidierten Öles erfolgt nach chemischen Standardmethoden, bevorzugt ist die Sulfonierung mit Schwefeltrioxid, Natriumbisulfit oder Schwefelsäure. Vorteilhaft bei diesem Verfahren ist, dass während der enzymatischen Peroxidation der Wasseranteil des Reaktionsgemisches auf einen Wert von etwa 30 % eingestellt werden kann, so dass das resultierende Gemisch enthaltend Wasser, Triglycerid und Sojamehl direkt mit Schwefeltrioxid oder Natriumbisulfit sulfoniert werden kann. Das resultierende Produkt ist eine partiell sulfonierte bzw. sulfatierte Triglycerid-Wasser Emulsion, die zusätzlich durch partiell sulfoniertes bzw. sulfatiertes Protein und Lipid insbesondere Phospholipid aus dem zugesetzten Sojamehl welches die Lipoxidasen zur Hydroperoxidation liefert, stabilisiert wird. Das so sulfonierte Sojamehl enthaltend sulfonierte bzw. sulfatierte Proteine, Lipide und Phospholipide besitzt zusätzlich emulgierende und lederfettende Eigenschaften und kann als stabile Wasser-Öl Emulsion im Gemisch mit dem sulfonierten, hydroperoxidierten Triglycerid direkt zur Lederfettung eingesetzt werden.

Sulfonierte Öle werden bisher chemisch in einem 2-Stufenprozess hergestellt (US 5529704; SU 1759835; EP 247490; DE 3617691; EP 564980). Die mehrfach ungesättigten Fischöle werden in der ersten Stufe geblasen. Hierbei wird bei hoher Temperatur Luft oder reiner Sauerstoff in das gerührte Fischöl eingeblasen. Sauerstoff wird dabei angelagert und ungesättigte Fischölhydroperoxide entstehen. Die Doppelbindungszahl im Öl nimmt bei dieser Reaktion ab. Die Verteilung von Fettsäuren in Fischöl aus Hering und Sardinen wird beispielhaft angegeben:

| **Fettsäure** | **C-Kettenstruktur** | **Heringsöl** | **Sardinenöl** |
|---|---|---|---|
| Palmitinsäure | C16 | 11-16% | 9-11 % |
| Palmitoleinsäure | C16:1 | 5-12 % | 10-15 % |
| Stearinsäure | C18 | 0-3 % | 1-3 % |
| Ölsäure | C18:1 | 8-15 % | 15-25 % |
| Linolsäure | C18:2 | 2-4 % | 3-8 % |
| Linolensäure | C18:3 | 0-2 % | 1-3 % |
| PUFAs (a) | C20:2-6 | 20-30 % | 15-30 % |
| PUFAs (b) | C22:3-6 | 10-28 % | 15-20 % |

Durch die benötigten hohen Reaktionstemperaturen in den herkömmlichen Verfahren werden während des Blasens verschiedene Nebenprodukte gebildet. Die gebildeten Hydroperoxide werden teilweise zu Ketonen umgewandelt und Glyceridketten werden an der angelagerten Hydroperoxidgruppe gespalten, so dass oxidierte kurzkettige Triglyceride entstehen. Durch die radikalische Bildung von Dialkylhydroperoxiden kommt es zusätzlich zur partiellen Polymerisation des Fischöls, was zu einem Anstieg der Viskosität im Hydroperoxid führt. Ab Peroxidzahlen von ∼ 200 nimmt die Bildung der Dialkylhydroperoxide bei herkömmlichen Verfahren stark zu, so dass technisch nur peroxidierte Fischöle mit Peroxidwerten von 400 - 500 zugänglich waren.

Das geblasene Fischöl wird nach der Peroxidierung erfindungsgemäß sulfoniert oder sulfatiert, wobei entweder Schwefeltrioxid in ein Fischöl-Hydroperoxid - Wasser Gemisch eingeleitet wird oder die Umsetzung mit einer wässrigen Natriumbisulfit-Aufschlämmung erfolgt. Sulfatierte Produkte sind über Schwefelsäure-Addition zugänglich. Die Umsetzung erfolgt bei erhöhten Reaktionstemperaturen.

In der Sulfonierungs- / Sulfatierungs-Reaktion werden sowohl die freien Hydroperoxide als auch die durch Umlagerung oder Kettenspaltung entstandenen Keto- und Aldehydverbindungen umgesetzt. Die Dialkylhydroperoxide werden dagegen nicht umgesetzt.

Die Sulfonate werden in einer Öl in Wasser Emulsion zur Lederfettung eingesetzt. Die negativ geladenen Sulfonate können in das Leder eindringen und dort mit den positiven Ladungen des Leders wechselwirken. Durch die Behandlung des Leders mit Sulfonaten, insbesondere Fischölsulfonat, wird das Leder weich und geschmeidig gemacht ohne Fettrückstände oder ein sensorisch fettiges Gefühl zu hinterlassen.

Die nach dem erfindungsgemäßen Verfahren erhaltenen Hydorperoxide stellen Vorstufen dar und eignen sich für die direkte Synthese von Produkten für die unterschiedlichen Verwendungen.

Ein weiterer Gegenstand der Erfindung ist daher die Verwendung von Hydroperoxiden, hergestellt nach dem erfindungsgemäßen Verfahren als Synthesevorstufe zur Herstellung von Lederfettungsmitteln, wobei die Hydroperoxide bevorzugt in Form von Triglyceriden hergestellt werden. Die im Sinne der Erfindung hergestellten enzymatisch hydroperoxidierten Öle, bevorzugt hochungesättigte Öle und/oder Fette sind geeignet chemisch hergestellte Hydroperoxide insbesondere zur Herstellung von Lederfettungsmitteln zu ersetzen. Zur Lederfettung werden seit langem sulfonierte Öle eingesetzt, wobei insbesondere Fischöl zur Anwendung kommt.

Ein weiterer Gegenstand der Erfindung ist ein Verfahren zur Herstellung von Hydroxyoctadecadiensäure (HODE) und/oder Hydroxyoctadecadiensäurederivaten, dadurch gekennzeichnet, dass Hydroperoxide, hergestellt nach dem erfindungsgemäßen Verfahren anschliessend selektiv reduziert werden, insbesondere durch Reaktion mit Natriumborhydrid. Die Reduktion mit Natriumborhydrid führt dazu, dass selektiv die Peroxidgruppe reduziert wird, dabei aber das konjugierte Doppelbindungssystem intakt bleibt.

Ein weiterer Gegenstand der Erfindung ist die Verwendung von Hydroperoxiden, hergestellt nach dem erfindungsgemäßen Verfahren als Synthesevorstufe zur Herstellung von 13-Hydroxyoctadecadiensäure (HODE) und 15-Hydroxyeicosatetraensäure (HETE) als freie Säure oder in derivatisierter Form zum Einsatz in kosmetischen Formulierungen. Bevorzugte Ausgangssubstrate für HODE oder HETE sind Linol- und Arachidonsäure, sowie deren Derivate und Fettsäuremethylester und Fettsäureethylester aus Distelöl.

Über den Einsatz von Linol- oder Arachidonsäure sind die Vorstufen der antiinflammatorisch wirkenden Produkte 13-HODE und 15-HETE darstellbar. Die freien Säuren können nicht direkt in kosmetischen Produkten eingesetzt werden. Ein Vorteil des erfindungsgemäßen Verfahrens ist es, dass auch direkt in kosmetischen Formulierungen einsetzbare Produkte für die Hydroperoxidierung zugänglich sind, hierzu gehören Derivate wie z.B. Ester oder Glyceride und Phophatidylglyceride. Diese Vorstufen sind zudem in hohen Substratkonzentrationen peroxidierbar. Die direkte Hydroperoxidierung von für die kosmetische Anwendung geeigneten Verbindungen zur Herstellung von 13-HODE und 15-HETE Derivaten stellt somit einen Teil der erfindungsgemäßen Lehre dar. 13-HODE und 15-HETE zeigen für die kosmetische Anwendung interessante Eigenschaften. 15-HETE wirkt antiinflammatorisch und 13-HODE reguliert beispielsweise die Maintenance der epidermalen Wasserbarriere. Topische Applikationen von 13-HODE führen zur Regenerierung von Hyperproliferationen und Dermatosen und reduzieren den transepidermalen Wasserverlust. Dabei wirkt 13-HODE inhibierend auf Protein Kinase C, die an Hyperproliferation und Tumor-Induktion beteiligt ist. 13-HODE zeigt dabei eine antagonistische Funktion zu Arachidonat-Produkten, insbesondere 12-HETE, und bewirkt eine Hemmung von Tumor Entstehung in der Haut (Fischer S.M. et al., 1996, Arachidonate has protumor-promoting action that is inhibited by linoleate in mouse skin carcinogenesis, J. Nutr. 126, 1099S-1104S).

Der Einsatz von 13-HODE in kosmetischen Formulierungen mit anti-psoriatischer Wirkung ist bereits in der WO 99/44585 beschrieben.

Ein weiterer Gegenstand der Erfindung ist die Verwendung von Hydroperoxiden, hergestellt nach dem erfindungsgemäßen Verfahren als Synthesevorstufe zur Herstellung von Phytoalexinen zum Einsatz in Pflanzenschutz-Produkten oder in kosmetischen und/oder pharmazeutischen Zubereitungen. Bevorzugte Ausgangssubstrate zur Herstellung von Phytoalexinen sind Linol- und Linolensäure, sowie deren Derivate.

Im Sinne der Erfindung versteht man unter Phytoalexinen Stoffe, die von Pflanzen aufgrund einer Infektion (postinfektionell) zur Abwehr des Schadorganismus - meist Pilze - gebildet werden. Die Akkumulation solcher Phytoalexine wird u. a. für die Resistenz bestimmter Pflanzen gegenüber pathogenen Pilzen verantwortlich gemacht. Damit lassen sich die Phytoalexine mit den Interferonen der Säugetiere vergleichen: beide sind wirtsspezifisch, werden auf einen äußeren Reiz hin gebildet, wirken aber ziemlich unspezifisch. Die Phytoalexine gehören sehr unterschiedlichen Stoffgruppen an wie beispielsweise (Beispiele in Klammern): Terpenoide und Sesquiterpenoide (Gossypol, Rishitin aus Kartoffeln, Capsidiol aus Paprikafrüchten), Isoflavonoide (Pisatin, Phaseolin, Sativan, Glyceol(I)in aus Sojabohnen), Furanoterpenoide (Ipomeamaron), Polyine (Safinol aus Saflor, Wyeron u. Wyeronsäure aus Saubohnen), Dihydrophenanthrene (Orchinol) oder Furocumarine (Xanthotoxin). Auslöser der Phytoalexine-Bildung sind nicht nur PilzInfektionen, sondern auch die Einwirkung von Bakterien, Viren, Nematoden, aber auch Verletzungen, Kälte, UV-Strahlung, Schwermetalle etc. Im einzelnen wird die Phytoalexine-Synthese z. B. bei Pilzinfektionen durch bestimmte Polysaccharide der Pilz-Zellwand eingeleitet.

Mit Hilfe des erfindungsgemäßen Verfahrens lassen sich Derivate von Linol- und Linolensäure wie z.B. Pflanzenöle oder Ester direkt in hohen Substratkonzentrationen hydroperoxidieren, so dass eine direkte weitere Umsetzung der Hydroperoxide mit Pflanzenextrakten ohne intermediäre Aufarbeitung des Produktgemisches zu Phytoalexin-Gemischen möglich ist.

Ein weitere Gegenstand der Erfindung ist die Verwendung von Hydroperoxiden, hergestellt nach dem erfindungsgemäßen Verfahren als Synthesevorstufe zur Herstellung der Aromakomponenten Hexenal, Hexenol, Nonadienal und Nonadienol. Bevorzugte Ausgangssubstrate für die Herstellung der Aromakomponenten sind Linol- und Linolensäure, sowie deren Derivate. Diese Aromastoffe zählen zu den pflanzlichen Stoffwechselprodukten basierend auf peroxidierte Fettsäuren.

Ein Vorteil des erfindungsgemäßen Verfahrens zur Herstellung der Aromakomponenten Hexenal, Hexenol sowie Nonadienal und Nonadienol ist der direkte Einsatz von Estern und Ölen mehrfach ungesättigter Linol- oder Linolensäure, was die Einsatzkonzentration der Substrate im Vergleich zu den beschriebenen Verfahren in WO9324644 und EP0911414 deutlich erhöht. In den beschriebenen Verfahren werden jeweils die freien Fettsäuren (beschrieben als Ölhydrolysat) zur Reaktion eingesetzt. Mit dem erfindungsgemäßen Verfahren kann daher die Raum-Zeit-Ausbeute an Aromakomponenten verbessert werden.

Ein weiterer Gegenstand der Erfindung ist die Verwendung von Hydroperoxiden, hergestellt nach dem erfindungsgemäßen Verfahren als Polymerisationsstarter in Lacken, Harzen und Coatings. Bevorzugte Ausgangssubstrate zur Herstellung von Polymerisationsstartern sind Leinöl, Tallöl oder hochungesättigte pflanzliche Öle. Die entsprechenden Hydroperoxide entstehen in herkömmlicher Weise bei der Lufttrocknung von Lacken (Firnis), Alkydharzen und Coatings und lösen die Polymerisation aus. Über direkte Zugabe der erfindungsgemäßen, enzymatisch hergestellten Hydroperoxide zum Oberflächenversiegelungsprodukt (2-Komponentenprodukt) kann die Trocknungszeit gezielt eingestellt werde. Die enzymatisch hergestellten Hydroperoxide sind bei niedrigen Temperaturen, bevorzugt eingefroren, lagerstabil.

Ein weiterer Gegenstand der Erfindung ist die Verwendung von Hydroperoxiden, hergestellt nach dem erfindungsgemäßen Verfahren als Synthesevorstufe zur Herstellung von 9- und 13-Hydroxystearinsäure, bevorzugt als Baustein zur Herstellung von Plastik oder als Inhaltsstoff in kosmetischen Produkten. Bevorzugte Ausgangssubstrate sind Linolsäure und deren Derivate.

Bei Einsatz von Linolsäure-haltigen Ölen ist dies mit einer hohen Produktausbeute durchführbar. Ein Vorteil dieses Verfahrens ist, dass Linolsäure-haltige Öle leichter zugänglich sind als Rizinolsäure-haltige Öle, die bisher zur Herstellung der analogen Säure 12-Hydroxystearinsäure eingesetzt werden.

Hydroxystearinsäure wird technisch aus Rizinusöl über Hydrierung und anschliessende Spaltung des Triglycerids gewonnen, wobei als Produkt 12-Hydroxystearinsäure entsteht. Verschiedene Verfahren beschreiben diesen Prozess, wobei die Hydrolyse des hydrierten Triglycerides entweder chemisch (Melanco C., 1993, Rev. Soc. Quim. Mex. 37, 66-69) oder enzymatisch (JP61139396) durchgeführt werden kann. Die 12-Hydroxystearinsäure findet in Form ihrer Salze, den 12-Hydroxystearaten Anwendung als Zwischenstufe zur Herstellung von Plastik oder als Inhaltsstoff in kosmetischen Produkten.

Über die Herstellung von Hydroperoxiden aus Linol- oder Linolensäure gefolgt von Hydrierung der ungesättigten Verbindungen sind die der 12-Hydroxystearinsäure verwandten Säuren 9- und 13-Hydroxystearinsäure zugänglich, die ein ähnliches Anwendungspotential haben.

Die nachfolgenden Beispiele erläutern die Gegenstände der Erfindung.

### Beispiele

### 1. Beispiel: Herstellung von Lipoxidase-Katalysator (am Beispiel von Sojabohnen)

### a) Herstellung eines wässrigen Lipoxidase-Extrakt

Sojabohnen (z.B. der Sorte Glycine max) wurden im Verhältnis 1 Teil zu 8 Teilen mit kaltem Wasser vermischt und als Gemisch 2 Minuten unter Zugabe von einem Teil Eis direkt vermahlen, bis eine Aufschlämmung aus Sojamehl in Wasser entstandt. Die Temperatur wurde beim Mahlen unter 40 °C gehalten. Diese wässrige Aufschlämmung war direkt zur enzymatischen Peroxidation einsetzbar.

### b) Herstellung eines angereicherten wässrigen Lipoxidase-Extraktes

Die in a) erhaltene wässrige Soja-Aufschlämmung wurde über ein Filtertuch von festen Bestandteilen abgepresst, eingefroren und anschliessend lyophilisiert. Der getrocknete Lipoxidase angereicherte Feststoffen wurde entweder direkt oder gelöst in Borat-Puffer pH 9 zur enzymatischen Peroxidierung eingesetzt.

### c) Herstellung eines angereicherten entölten Lipoxidase-Extraktes

Sojabohnen (z.B. der Sorte Glycine max) wurden mit flüssigem Stickstoff gekühlt und mit einer Mühle zu Pulver gemahlen. Das Pulver wurde zweimal mit dem 3fachen Überschuss an Aceton gewaschen und über ein Filtertuch gepresst. Restliches Aceton wurde aus dem entölten Sojapulver bei Raumtemperatur im Vakuum abgezogen. Das Sojapulver wurde mit einem Borat-Puffer pH 9 aufgeschlämmt. Die erhaltene Aufschlämmung war direkt zur enzymatischen Peroxidation einsetzbar.

Alternativ wurde die so erhaltene wässrige Aufschlämmung über ein Filtertuch gepresst und der klare Überstand eingefroren und anschliessend lyophilisiert. Das so erhaltene Pulver konnte direkt oder in Wasser konzentriert gelöst zur Peroxidation eingesetzt werden.

### 2. Beispiel: Bestimmung der Lipoxidase-Enzymaktivität

Die Bestimmung der Lipoxidase-Enzymaktivität erfolgte nach der Methode von Samuelsen (M. Hamberg & B. Samuelsen; J. Biol. Chem. 242 (1967), 5329).

1 Teil Lipoxidase-haltiges Enzymprodukt wurden mit 9 Teilen Borat-Puffer (Konzentration 200 mM, pH = 9,0), gemixt und zentrifugiert. Der klare Überstand nach Zentrifugation wurde 1 : 10 mit Borat-Puffer verdünnt. 10 µl des verdünnten Extrakts wurden mit 990 µl Reaktions-Puffer gemischt und die Absorptionszunahme bei 234 nm wird UV-photospektometrisch bestimmt. Detektiert wurde die Bildung von konjugierten Doppelbindungen im Substrat Linolsäure.

Der Reaktions-Puffer wurde wie folgt hergestellt: 10 µl Linolsäure wurden mit 10 µl Ethanol gemischt und 10 ml Wasser wurden unter Rühren zugegeben. Anschließend wurden 50 ml Borat-Puffer (Konzentration 200 mM, pH = 9,0) zugegeben und der Reaktionspuffer wurde bei - 20 °C gelagert.

Eine Unit (Enzymaktivitätseinheit) entspricht definitionsgemäß einem Absorptionsanstieg bei 234 nm von 0,001 pro min bei pH 9.0 und 25 °C mit einer Konzentration von 0,011 % (v/v) Linolsäure bei 1 cm Lichtweg durch die Reaktionslösung. Dies entspricht der Oxidation von 0,12 µmol Linolsäure pro min.

### 3. Beispiel: Enzymatische Peroxidation von linolsäurehaltigem Sonnenblumenöl

250 Teile Sonnenblumenöl und 50 Teile Wasser dest wurden gemischt und mit NaOH auf pH 9 eingestellt. 50 Teile entölte Sojaaufschlämmung in Borat-Puffer pH 9 und 0,25 Teile Terminox 50L (Novo Nordisk) wurden zugegeben und verrührt. Über 28 h wurden kontinuierlich 42 Teile 30% Wasserstoffperoxid-Lösung und 280 Teile Sojaextrakt / Terminox Gemisch über Pumpen zudosiert.

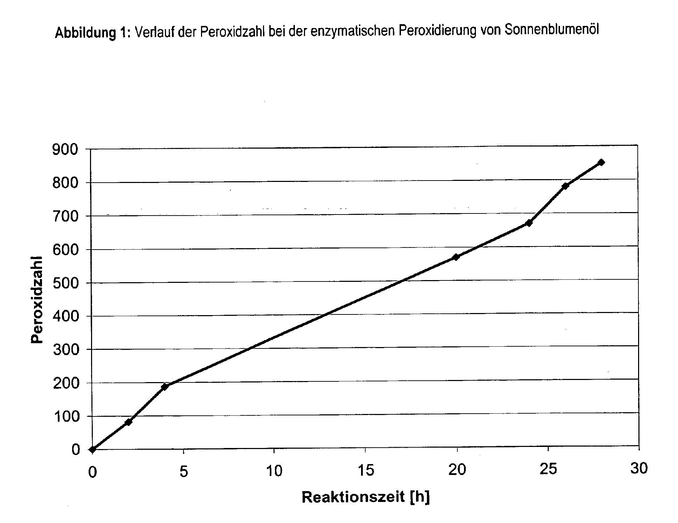

Zur Bestimmung des Peroxid-Gehaltes wurde die wässrige von der Ölphase über Zentrifugation getrennt. Über 28 h war ein kontinuierlicher Anstieg der Peroxidzahl zu beobachten (Abbildung 1).

### 4. Beispiel: Enzymatische Peroxidation von Fischöl und Vergleich zur chemischen Peroxidation

500 Teile Fischöl wurden mit 3 Teilen 30% NaOH gemischt und gerührt. Nach 5 min wurden 50 Teile wässrige Sojamehl Aufschlämmung und 1 Teil 30 % NaOH zugegeben. Nach 30 min erfolgte eine weitere Zugabe von 50 Teilen Soja-Aufschlämmung und 3 Teilen Terminox 50L (Novo Nordisk). Anschließend wurden 4 Teile 30% Wasserstoffperoxid-Lösung kontinuierlich über 10 h zugepumpt. Während dieser Zeit wurden alle 30 min 12 Teile Soja-Aufschlämmung zugegeben. Nach 10 h wurde die Peroxidation bei einer Peroxidzahl von 200 beendet.

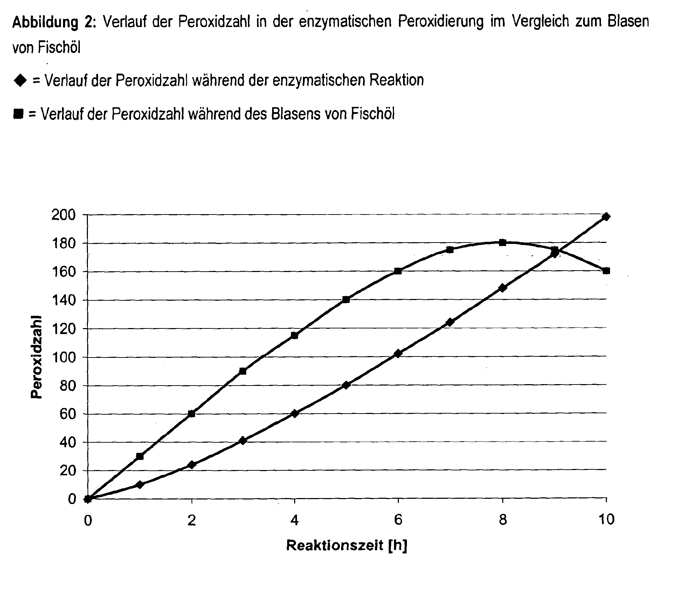

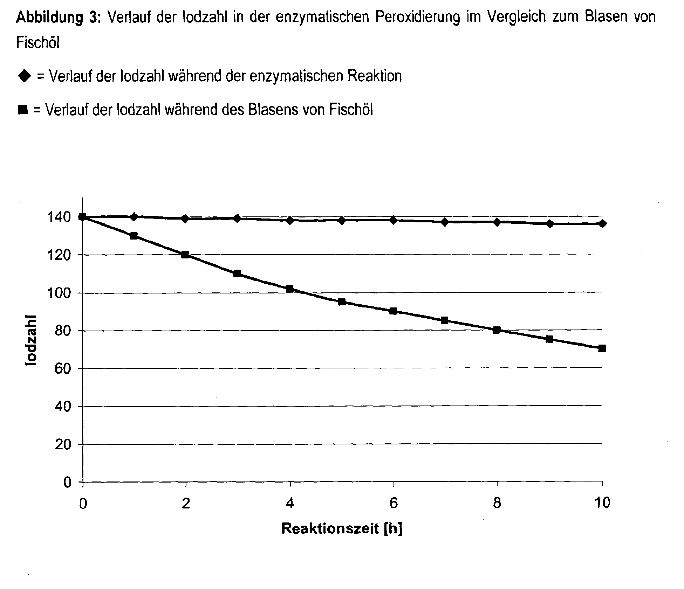

Aufgezeichnet wurde der typische Verlauf der Peroxidierung enzymatisch nach Beispiel 2 verglichen mit der chemischen Peroxidation (Blasen) von Fischöl in der Hitze (Abbildung 2). Während beim Blasen von Fischöl ein Maximum der Peroxidzahl durchlaufen wird, stieg die Peroxidzahl nach dem enzymatischen Verfahren stetig an. Weiterhin aufgezeigt ist der typische Verlauf der lodzahl (Anteil Doppelbindungen) während der Peroxidierung nach dem enzymatischen Verfahren im Vergleich zum Blasen des Fischöls (Abbildung 3). Die lodzahl fiel im enzymatischen Verfahren kaum ab.

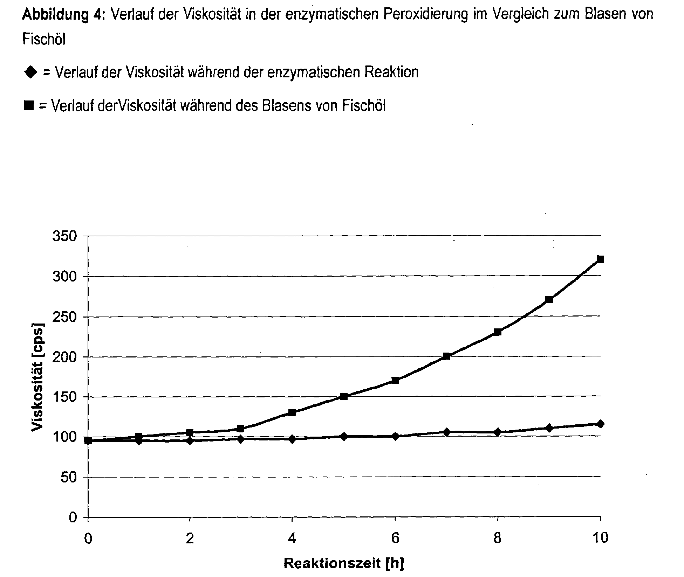

Die Viskosität nahm während der enzymatischen Peroxidation im Zeitraum von 10 h nicht signifikant zu. Beim Blasen von Fischöl war ein starker Anstieg in der Viskosität zu beobachten, der abhängig von der Blastemperatur ist (typischer Kurvenlauf: Abbildung 4).

### 5. Beispiel: Herstellung von Fettsäurederivaten aus den entsprechenden Triglyceriden

Herstellung von Fettsäuremethylester aus Distelöl: 1150 g Distelöl aus *Echinops sphaerocephalus* wurde in einem Rundkolben für 30 min bei 120 °C und 30 mbar getrocknet. Das Öl wurde auf 60 °C abgekühlt, mit 137 g Methanol und 21,5 g Natriummethylat versetzt. Bei 70 °C wurde für 2 h unter Stickstoff-Begasung gerührt. Nach Separation der Phasen wurde die obere Phase abgetrennt und zur Nachveresterung mit 20,8 g Methanol unter obigen Bedingungen gerührt. Nach Phasenseparation wurde der entstandene Ester zweimal mit heißem Wasser gewaschen, bei 120 °C und 30 mbar für 30 min getrocknet und anschliessend bei einer Temperatur von 186 °C überdestilliert.

Die Herstellung von Ethylestern funktioniert analog über Zugabe von Ethanol und Kaliumethylatlösung.

Die gaschromatographischen Analysen wurden mit einer HP-1 Säule (Agilent) in einem Temperaturgradient von 150 - 300 °C durchgeführt. 30 mg der Probe wurde mit 2 ml Trimethylsilylfluoroacetamid/N-Methyl-Trimethylsilylfluoroacetamid-Lösung und 2 ml Pyridin versetzt und 30 min bei 80 °C inkubiert. 1 µl der so vorbereiteten Lösung wurde in den Gaschromatographen injiziert.

Ergebnis: Nach gaschromatographischer Analyse wurde das Öl quantitativ in die entsprechenden Ester umgesetzt. Die Fettsäurezusammensetzung der gewonnenen Ester entspricht der des Substrates.

| Fettsäure | Distelöl [%] | Fettsäuremethylester [%] | Fettsäureethylester [%] |
|---|---|---|---|
| Palmitinsäure | 3-10 | 3,9 | 6,2 |
| Stearinsäure | 1-5 | 2,0 | 2,4 |
| Ölsäure | 10-20 | 17,3 | 13,6 |
| Linolsäure | 70-80 | 76,6 | 72,1 |

### 6. Beispiel: Enzymatische Peroxidation von Fettsäureethylester basierend auf der Zusammensetzung von Sonnenblumenöl

250 Teile Fettsäureethylester und 50 Teile Wasser dest wurden gemischt und mit NaOH auf pH 9 eingestellt. 100 Teile Sojaaufschlämmung in Borat-Puffer pH 9 und 0,25 Teile Terminox 50L (Novo Nordisk) wurden zugegeben und verrührt. Über 7 h wurden kontinuierlich 17,5 Teile 30% Wasserstoffperoxid-Lösung über eine Pumpe zudosiert.

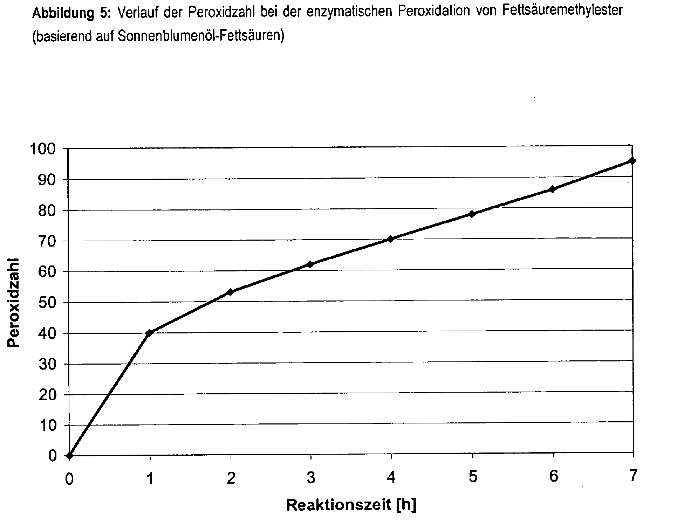

Zur Bestimmung des Peroxid-Gehaltes wurde die wässrige von der Ölphase über Zentrifugation getrennt. Über 7 h war ein kontinuierlicher Anstieg der Peroxidzahl zu beobachten (Abbildung 5).

### 7. Beispiel: Enzymatische Peroxidation von Fettsäuremethylester basierend auf der Zusammensetzung von Distelöl

In einen 1 Liter Kolben wurden 500 ml Puffer (Borat, 20 mM, pH 9,0), 100 ml Fettsäuremethylester basierend auf Distelöl, 100 ml filtrierter Lipoxidaseextrakt aus Sojabohnen und 15 ml Katalase vorgelegt. Die Reaktion wurde über Zudosierung von 30 %iger Wasserstoffperoxidlösung mit einem Volumenstrom von 0,16 ml/min gestartet. Zeitgleich wurde auch eine kontinuierliche Dosierung von Lipoxidaselösung mit 0,16 ml/min sowie Katalaselösung mit 9 µl/min gestartet. Die Reaktion wurde unter kontinuierlichem Rühren und unter Kühlung durchgeführt, wobei eine Temperatur von konstant 25 °C eingestellt wurde.

Während der Reaktion wurden Proben entnommen und der Gehalt an Wasserstoffperoxid in der wässrigen Phase mit Einwegteststäbchen (Merck) überprüft. Bei einem Anstieg des Wasserstoffperoxidgehaltes in der wässrigen Phase von > 2 mg/l wurde der Volumenstrom Katalase kurzfristig erhöht. Die Peroxidzahl der Ölphase wurde nach Wheeler bestimmt (Beispiel 10).

Nach Abbruch der Synthesereaktion wurden die Phasen über 30 minütige Zentrifugation bei 10.000 Upm und 4 °C getrennt, die obere Hydroperoxidphase wurde abgenommen und bei - 20 °C gelagert.

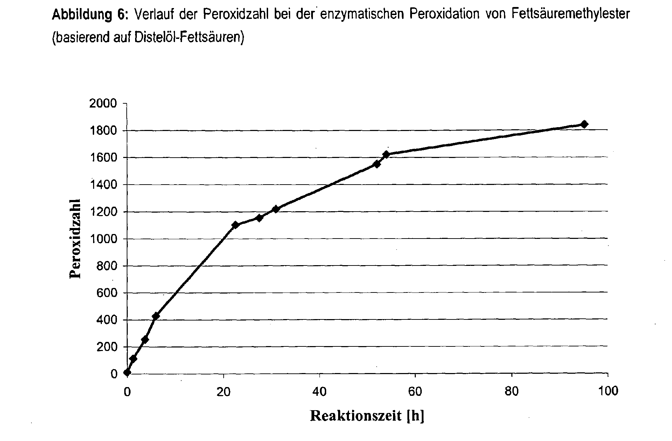

Ergebnis: Ein kontinuierlicher Anstieg der Peroxidzahl im Produkt wurde festgestellt. Nach 95 h Reaktionszeit wurde eine Peroxidzahl von 1840 festgestellt. Der Anstieg der Peroxidzahl in Abhängigkeit der Zeit ist in Abbildung 6 dargestellt.

### 8. Beispiel: Enzymatische Peroxidation von Fettsäureethylester basierend auf der Zusammensetzung von Distelöl in Anwesenheit und Abwesenheit von Proteaseinhibitor

In einen 1 Liter Kolben wurden 500 ml Puffer (Borat, 20 mM, pH 9,0), 100 ml Fettsäureethylester basierend auf Distelöl, 100 ml filtrierter Lipoxidaseextrakt aus Sojabohnen und 15 ml Katalase vorgelegt. Die Reaktion wurde über Zudosierung von 30 %iger Wasserstoffperoxidlösung mit einem Volumenstrom von 0,16 ml/min gestartet. Zeitgleich wurde auch eine kontinuierliche Dosierung von Lipoxidaselösung mit 0,16 ml/min sowie Katalaselösung mit 9 µl/min gestartet. Die Reaktion wurde unter kontinuierlichem Rühren und unter Kühlung durchgeführt, wobei eine Temperatur von konstant 25 °C eingestellt wurde.

Zwei Ansätze wurden parallel gestartet. In einen der beiden Ansätze wurden bei Reaktionsstart zusätzlich 2 ml Proteaseinhibitor Cocktail (Sigma Aldrich) zugesetzt.

Während der Reaktion wurden Proben entnommen und der Gehalt an Wasserstoffperoxid in der wässrigen Phase mit Einwegteststäbchen (Merck) überprüft. Bei einem Anstieg des Wasserstoffperoxidgehaltes in der wässrigen Phase von > 2 mg/l wurde der Volumenstrom Katalase kurzfristig erhöht. Die Peroxidzahl der Ölphase wurde nach Wheeler bestimmt (Beispiel 10).

Nach Abbruch der Synthesereaktion wurden die Phasen über 30 minütige Zentrifugation bei 10.000 Upm und 4 °C getrennt, die obere Hydroperoxidphase wurde abgenommen und bei - 20 °C gelagert.

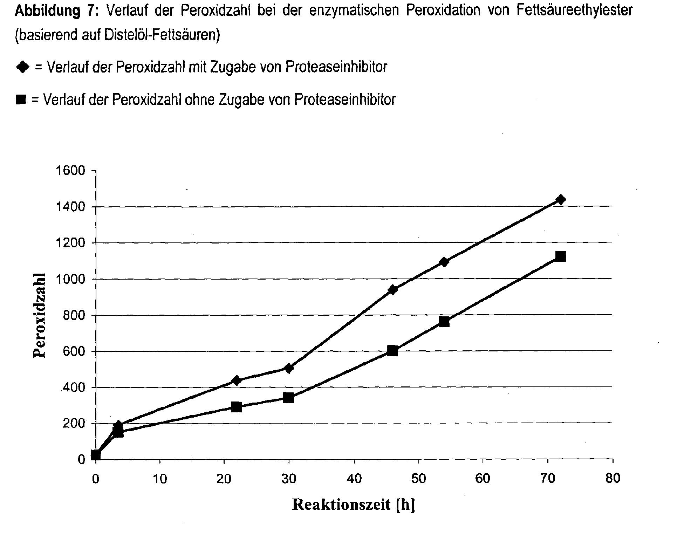

Ergebnis: Ein kontinuierlicher Anstieg der Peroxidzahl im Produkt wurde festgestellt. Nach 71 h Reaktionszeit wurde eine Peroxidzahl von 1440 beim Ansatz mit Proteaseinhibitor und eine Peroxidzahl von 1120 beim Ansatz ohne Proteaseinhibitor festgestellt. Der Anstieg der Peroxidzahl in Abhängigkeit der Zeit ist in Abbildung 7 dargestellt.

### 9. Beispiel: Lagerstabilität von Sonnenblumenöl-Hydroperoxiden

Hydroperoxidiertes Sonnenblumenöl mit einer POZ-Zahl von 850 (aus Beispiel 2) wurde bei verschiedenen Temperaturen für 6 Wochen gelagert.

| Ergebnis: | Temperatur | POZ-Zahl |
|---|---|---|
| | -30 °C | 750 |
| | -6 °C | 735 |
| | 25 °C | 460 |

### 10. Beispiel: Bestimmung der Peroxidzahl in peroxidiertem Triglycerid bzw. in peroxidierten Fettsäurederivaten (nach Wheeler)

Die Peroxidzahl ist ein Maß für den Gehalt an gebundenen Peroxidgruppen angegeben in mmol Peroxid pro kg. Die Bestimmung erfolgte nach dem Verfahren von Wheeler (R.F. Paschke & D.H. Wheeler, Oil and Soap 21 (1944), 52).

5 g Fischöl wurden in 30 ml Eisessig-Chloroform-Gemisch (3:2) gelöst und 0,5 ml gesättigte Kaliumiodid-Lösung wurden zugegeben. Nach 1 min wurden 50 ml Wasser zugesetzt und das ausgeschiedene lod wurde mit 0,1 n Natriumthiosulfat-Lösung und Stärke als Indikator titriert.

Die Bestimmung der Peroxidzahl von Fettsäurederivaten wird analog zur Bestimmung der Peroxidzahl von Fischöl durchgeführt.

Die Peroxidzahl errechnet sich nach: POZ = (a-b)*N*1000/E

POZ = Peroxidzahl, a = ml Thiosulfat-Lösung, b = ml Thiosulfat-Lösung im Blindversuch, N = Normalität der Natriumthiosulfat-Lösung, E = Einwaage in g

### 11. Beispiel: Bestimmung der lodzahl in ungesättigten Verbindungen (nach Kaufmann)

Die lodzahl als Maß für den Gehalt an ungesättigten Verbindungen gibt die Teile an lod, die 100 Teile Fett (an den Doppelbindungen) unter den genannten Bedingungen zu binden vermögen.

Fischöl (0,5 g) wurde in 10 ml Dichlormethan gelöst. 20 ml Brom-Lösung (15 Teile Natriumbromid in 100 Teilen Methanol) wurden addiert. Nach 15 min wurden 15 ml Kaliumiodid-Lösung (10 %) zugegeben und es wurde gegen 0,1 n Natriumthiosulfatlösung mit Stärke als Indikator titriert.

Die Bestimmung der Peroxidzahl von Fettsäurederivaten wird analog zur Bestimmung der Peroxidzahl von Fischöl durchgeführt. Der Bestimmung der lodzahl wurde in gleicher Form auch mit den konjugiert ungesättigten peroxidierten Verbindungen bzw. den über Reduktion hergestellten konjugiert ungesättigten Hydroxyverbindungen durchgeführt.

Die lodzahl errechnete sich nach: IZ = (b-a)*1.269/E

IZ = lodzahl, a = ml Thiosulfat im Versuch, b = ml Thiosulfat im Blindversuch, E = Einwaage in g

### 12. Beispiel : Bestimmung der Hydroxylzahl

Die Bestimmung der Hydroxylzahl erfolgt nach der Methode DGF C-V 17a, wobei die Säurezahl mit in die Hydroxylzahl einfliesst. Die Säurezahl wird bestimmt nach der Methode C-V 2.

Bestimmung der Säurezahl: 1 g Probe wurden in einen 300 ml Erlenmeyerkolben eingewogen, in 40 ml Ethanol gelöst und mit einigen Tropfen Phenophtalein als Indikator versehen. Dann wurde bis zum Farbumschlag von farblos nach rosa mit 0,1 molarer Kalilauge titriert.

Bestimmung der Hydroxylzahl: 5 g Probe wurden in einen 300 ml Erlenmeyerkolben eingewogen und mit 10 ml Acetylierungslösung (10 % Essigsäureanhydrid, 90 % Pyridin) versetzt. Der verschlossene Kolben wurde für 1 h bei 105 °C erhitzt, nach Abkühlen wurde 1 ml Wasser zugegeben und erneut für 10 min bei 105 °C inkubiert. Nach Abkühlen wurden 20 ml Ethanol und einige Tropfen Phenophtalein zugegeben und mit 0,5 M KOH wird von farblos nach rosa titriert.

Die Säurezahl errechnet sich nach: SZ = (a-b)*M*t/E
SZ = Säurezahl, a = Verbrauch KOH in ml im Versuch; b = Verbrauch KOH in ml im Blindversuch; t = Titer der KOH in mo/l; M = Molare Masse KOH; E = Einwaage Probe in g

Die Hydroxylzahl errechnet sich nach: OHZ = (b-a)*M*t/E + SZ
OHZ = Hydroxylzahl; SZ = Säurezahl, a = Verbrauch KOH in ml im Versuch; b = Verbrauch KOH in ml im Blindversuch; t = Titer der KOH in mo/l; M = Molare Masse KOH; E = Einwaage Probe in g

### 13. Beispiel : Sulfonierung des enzymatisch peroxidierten Fischöls

Das peroxidierte Fischöl-Wasser-Soja Gemisch wurde direkt zur Sulfonierung eingesetzt. Das Gemisch wurde auf 80 °C erhitzt, dann wurden 12 % festes Natriumbisulfit unter Rühren addiert. Dabei war ein Temperaturanstieg auf 90 - 95 °C zu beobachten. Die Sulfonierungs-Reaktion wurde unter Energiezufuhr 3 h bei 85 - 95 °C durchgeführt. Nach Abkühlen des sulfonierten Produktes wurde von unlöslichem Material abfiltriert.

### 14. Beispiel : Selektive Reduktion von peroxidierten Fettsäurederivaten zu Hydroxyoctadecadiensäurederivaten

10 ml Hydroperoxid (peroxidierter Fettsäuremethylester bzw. peroxidierter Fettsäureethylester) wurde in 200 ml kaltem Methanol gelöst. Unter Eiskühlung wurde portionsweise 1 g Natriumborhydrid zugegeben und 1 h unter Eiskühlung nachgerührt. Anschliessend wurden zum Abbruch der Reaktion 100 ml Wasser zugegeben und der reduzierte Ester wurde zweimal mit je 100 ml Chloroform extrahiert. Die Chloroformphase wurde abdestilliert, die zurückbleibende klare Flüssigkeit stellte das Produkt Hydroxyoctadecadiensäureester dar.

Ergebnis: Zur Reduktion wurde ein Fettsäuremethylester basierend auf Distelöl mit einer POZ von 1360 und ein Fettsäureethylester basierend auf Distelöl mit einer POZ von 1080 eingesetzt. Folgende nasschemische Kennzahlen wurden ermittelt:

| | Fettsäuremethylester | Fettsäureethylester |
|---|---|---|
| Vor Reduktion | | |
| POZ | 1360 | 1080 |
| OHZ | 13 | 10 |
| IZ | 136 | 135 |
| Nach Reduktion | | |
| POZ | 26 | 30 |
| OHZ | 49 | 45 |
| IZ | 136 | 135 |

Aus den nasschemischen Kenzahlen ist zu erkennen, dass die Reduktion mit Natriumborhydrid selektiv die Peroxidgruppe reduziert, dabei aber das konjugierte Doppelbindungssystem intakt lässt. Belegt wird dies durch die konstante IZ der Proben bei sinkender POZ-Zahl und entsprechend ansteigender OHZ-Zahl.

### 15. Beispiel : Lipase katalysierte Hydrolyse von peroxidierten Fettsäurederivaten bzw. Hydroxyoctadecadiensäurederivaten in die entsprechenden freien Säuren

1 ml peroxidierter Fettsäureester (Methylester oder Ethylester) bzw. 1 ml Hydroxyoctadecadiensäureester wurden mit 9 ml destilliertem Wasser versetzt. Dazu wurde 1 ml einer Flüssigpräparation von *Candida antarctica* B Lipase (Novozym 525) gegeben und die Hydrolysereaktion wurde unter Rühren bei Raumtemperatur über 15 h durchgeführt. Die Phasen wurden über 5 minütige Zentrifugation bei 14.000 Upm getrennt. Die obere, das jeweilige Spaltprodukt enthaltende Phase wurde abgenommen und bei - 20 °C gelagert.

### 16. Beispiel : Chromatographischer Nachweis der gebildeten peroxidierten Fettsäuren bzw. Hydroxyoctadecadiensäuren

Die chromatographische Analyse der gebildeten peroxidierten Fettsäureester bzw der entsprechenden reduzierten Fettsäureester erfolgte über HPLC-Vergleich mit Eichsubstanzen. Da Eichsubstanzen lediglich in Form der freien Säuren erhältlich sind, wurde der Vergleich mit den nach Beispiel 15 hydrolysierten Verbindungen durchgeführt. Bei der milden Lipase katalysierten Hydrolyse ist keine Zersetzung der Proben zu erwarten.

Als Referenzsubstanzen zur Analyse der peroxidierten Fettsäuren wurden 9(S)-Hydroperoxyoctadeca-10E,12Z-Diensäure (ICN) und 13(S)-Hydroperoxyoctadeca-9Z,11E-Diensäure (ICN) eingesetzt. Als Referenzsubstanzen zur Analyse der reduzierten Fettsäurehydroperoxide wurden 9-Hydroxyoctadeca-10E,12Z-Diensäure (ICN) und 13-Hydroxyoctadeca-9Z,11E-Diensäure (ICN) eingesetzt.

Die Auftrennung der Substanzgemische erfolgte mit einer Source 5RPC ST 4.6/150 Reversed Phase Säule (Pharmacia) in einem Gradienten aus Acetonitril und 0,1 %iger Phosphosäure. Die Detektion der Produkte wurde simultan bei 234 nm, 190 nm und 200 nm durchgeführt. 5 mg der zu analysierenden Substanz wurden in 2 ml Acetonitril gelöst, davon wurden jeweils 50 µl in die HPLC injiziert.

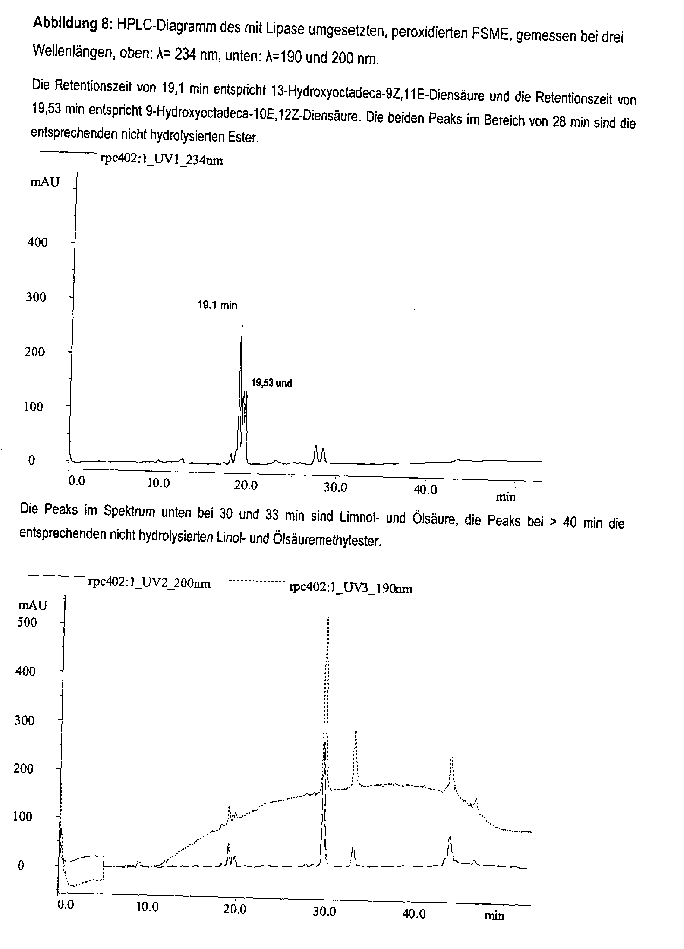

Ergebnis: Die 9- und 13-Hydroxy- bzw Hydroperoxyverbindungen konnten im Laufmittelgradienten getrennt werden. Die über Lipoxidase hergestellten und über Lipase hydrolysierten Produkte stimmten in ihrer Retentionszeit mit den Referenzsubstanzen überein. Ebenso wurde eine Übereinstimmung der reduzierten Verbindungen mit den Referenzsubstanzen erhalten (Abbildung 8). Weitere Verbindungen mit konjugierten Doppelbindungssystemen konnten nicht detektiert werden. Über die Lipoxidase katalysierte Peroxidierung wurden Substanzgemische erhalten, die einen Anteil des 13-Hydroperoxids von > 60 % bezogen auf alle Hydroperoxide enthalten.

## Patentansprüche

1. Verfahren zum Gewinnen von Hydroperoxiden aus einem nativen Fett oder Öl enthaltend ungesättigte Fettsäuren, oder ungesättigte Fettsäuren oder deren Derivate und/oder deren Mischungen, **dadurch gekennzeichnet, dass**
a) das native Fett oder Öl enthaltend ungesättigte Fettsäuren, oder ungesättigte Fettsäuren oder deren Derivate und/oder deren Mischungen unter katalytischer Einwirkung von Lipoxidase in Gegenwart von Katalase bei einer Temperatur zwischen 5 und 50°C peroxidiert werden, wobei die Katalase aus zudosiertem Wasserstoffperoxid Sauerstoff zur Peroxidation in situ produziert und
b) die wässrige Phase mit den Enzymen von der Ölphase gegebenenfalls abgetrennt wird
c) das so erhaltene Produkt gegebenenfalls chemisch transformiert und gegebenenfalls anschliessend konfektioniert wird.

2. Verfahren nach Anspruch 1, **dadurch gekennzeichnet, dass** die eingesetzte Lipoxidase aus Sojabohnen und/oder aus Mikroorganismen und/oder über rekombinante Expression aus Mikroorganismen gewonnen wird.

3. Verfahren nach Anspruch 1 und/oder 2, **dadurch gekennzeichnet, dass** die eingesetzte Katalase ausgewählt ist aus der Gruppe, die gebildet wird von Katalase aus Mikroorganismen, bevorzugt aus Aspergillus niger und/oder Micrococcus lysodeikticus.

4. Verfahren nach einem der Ansprüche 1 bis 3, **dadurch gekennzeichnet, dass** die eingesetzten Derivate ungesättigter Fettsäuren Ester dieser Fettsäuren darstellen, bevorzugt Methyl und/oder Ethylester und/oder Glyceride.

5. Verfahren nach einem der Ansprüche 1 bis 3, **dadurch gekennzeichnet, dass** die eingesetzten nativen Fette oder Öle und die ungesättigten Fettsäuren ausgewählt sind aus der Gruppe, die gebildet wird von Fischöl, Sonnenblumenöl, Tallöl, Leinöl, Sesamöl, Sojaöl, Mohnöl, Mandelöl, Maiskeimöl, Palmöl, Rapsöl, Reiskeimöl, Erdnussöl, Baumwollsaatöl, Linolsäure, Linolensäure, Arachidonsäure, Docosahexaensäure, Elaeostearinsäure, Sorbinsäure und Clupanodonsäure.

6. Verfahren nach Anspruch 1, **dadurch gekennzeichnet, dass** es sich bei dem Trennverfahren im Schritt b) um Phasenseparation über eine Zentrifuge oder einen Tellerseparator handelt.

7. Verfahren zur Herstellung von Sulfonaten zur Lederfettung, **dadurch gekennzeichnet, dass** Hydroperoxide, hergestellt nach einem Verfahren gemäß Anspruch 1 bis 6 anschliessend sulfoniert werden durch Reaktion mit Sulfiermitteln ausgewählt aus der Gruppe, die gebildet wid aus Schwefeltrioxid, Natriumbisulfit, Schwefelsäure, Oleum, Chlorsulfonsäure und Amidosulfonsäure.

8. Verfahren zur Herstellung von Hydroxyoctadecadiensäure (HODE) und/oder Hydroxyoctadecadiensäurederivaten, **dadurch gekennzeichnet, dass** Hydroperoxide, hergestellt nach einem Verfahren gemäß Anspruch 1 bis 6 anschliessend selektiv reduziert werden, insbesondere durch Reaktion mit Natriumborhydrid.

9. Verwendung von Hydroperoxiden, hergestellt nach einem Verfahren gemäß Anspruch 1 bis 6 als Synthesevorstufe zur Herstellung von Lederfettungsmitteln, wobei die Hydroperoxide bevorzugt in Form von Triglyceriden hergestellt werden.

10. Verwendung von Hydroperoxiden, hergestellt nach einem Verfahren gemäß Anspruch 1 bis 6 als Synthesevorstufe zur Herstellung von 13-Hydroxyoctadecadiensäure (HODE) und 15-Hydroxyeicosatetraensäure (HETE) als freie Säure oder in derivatisierter Form zum Einsatz in kosmetischen Formulierungen.

11. Verwendung von Hydroperoxiden, hergestellt nach einem Verfahren gemäß Anspruch 1 bis 6 als Synthesevorstufe zur Herstellung von Phytoalexinen zum Einsatz in Pflanzenschutz-Produkten oder in kosmetischen und/oder pharmazeutischen Zubereitungen.

12. Verwendung von Hydroperoxiden, hergestellt nach einem Verfahren gemäß Anspruch 1 bis 6 als Synthesevorstufe zur Herstellung der Aromakomponenten Hexenal, Hexenol, Nonadienal und Nonadienol.

13. Verwendung von Hydroperoxiden, hergestellt nach einem Verfahren gemäß Anspruch 1 bis 6 als Polymerisationsstarter in Lacken, Harzen und Coatings.

14. Verwendung von Hydroperoxiden, hergestellt nach einem Verfahren gemäß Anspruch 1 bis 6 als Synthesevorstufe zur Herstellung von 9- und 13-Hydroxystearinsäure bevorzugt als Baustein zur Herstellung von Plastik oder als Inhaltsstoff in kosmetischen Produkten.
